# EUROPEAN PATENT APPLICATION

(11) **EP 4 732 846 A2**
(43) Date of publication of application: **29.04.2026**
(21) Application number: 26163923.1
(22) Date of filing: 03.04.2025
(51) Int. Cl.: A61K 45/06

(54) **HYALURONIC ACID-BASED COMPOSITIONS FOR USE IN DRY EYE TREATMENT**

(30) Priority: 03.04.2024 US 202463574196 P
(62) Divisional of application: 25719848.1
(71) Applicant: Nordic Group B.V., 2132 WT Hoofddorp (NL)
(72) Inventor: HERBAGE, Benjamin, 69005 Lyon (FR); PACKER, Mark, 80521 Massachusetts (US)
(74) Representative: August Debouzy

(57) **Abstract**

New crosslinked hyaluronic acid gels are provided and use of same as canalicular occlusive devices and to treat dry eye disease. In one aspect, a composition for ocular administration is provided comprising a therapeutically effective amount of 1) crosslinked hyaluronic acid and 2) uncrosslinked hyaluronic acid, wherein the composition has i) a cohesivity value of at least 3 mg according to the drop weight method and ii) a G' elastic modulus of less than 400 Pa.

## Description

The present application claims the benefit of U.S. provisional application no. 63/574,196 filed April 3, 2024, which is incorporated by reference herein in its entirety.

### FIELD

In one aspect, new compositions and methods of use of crosslinked hyaluronic acid including as canalicular occluder for treating diseases of the eye, especially for treating dry eyes.

### BACKGROUND

Dry eye syndrome (DES) is a common condition that affects millions of people worldwide. A proper tear film is essential for maintaining eye lubrication and clear vision. Symptoms of DES include foreign body sensation, burning, irritation, pain, and sensitivity to light.

The management of dry eyes is one of the most common disorders treated in ophthalmic practice. Initial treatment of DES consists of medical therapy by replacing moisture with artificial tears and ointment. Punctal plugs are a common second-line treatment, and they function by blocking the outflow of tears from the eye. They have the advantage of using the patients' own tears to maintain the lubrication of the eye.

Plugs are made from a variety of materials, and there are several inherent problems with the current plug designs. Silicone plugs use a cap to hold the plug-in place which sits above the lid margin, so that it does not slip into the deeper tear duct. The plug cap can rub on the surface of the eye causing ocular irritation. Sizing the correct plug can be challenging, and the plug can occasionally be lost if it is not properly fitted.

Intracanalicular plugs have the potential benefit of being placed below the surface of the lid thereby avoiding rubbing directly on the eye, but they can act as a foreign body and cause canaliculitis.

It would be desirable to have new materials and methods for treatment of dry eye syndrome.

### SUMMARY

We now provide new hyaluronic acid (HA) compositions and treatment methods for dry eye syndrome.

We have demonstrated in human subjects therapeutic efficacy of the compositions disclosed herein, including human patients suffering from dry eye syndrome.

We also have demonstrated long-term efficacy in human subjects, including therapeutic benefit in excess of 3, 4 or 5 months after administration of a composition to a subject. Human subjects evaluated up to 6 months after administration of a composition as disclosed herein continued to demonstrate effective therapeutic benefit.

As further disclosed, the present compositions of crosslinked hyaluronic acid (HA) and free (uncrosslinked) bnchyaluronic acid (HA) have been successfully evaluated in a randomized, double masked human clinical study of patients suffering from dry eye syndrome where the patient's canaliculus is occluded with a present composition compared to inter alia a gel of polyvinylpyrrolidone that contains no free (uncrosslinked) HA. The present composition comprising an admixture of both crosslinked hyaluronic acid (HA) and free (uncrosslinked) hyaluronic acid (HA) demonstrated superior and long-lasting therapeutic benefit.

Thus, in one aspect, the present compositions comprise both crosslinked and free (uncrosslinked) hyaluronic acid (HA).

In a further aspect, the present composition may be selected and used based on displaying high cohesivity for occluding the canaliculus.

In a further preferred aspect, the present composition may be selected and used based on displaying high cohesivity for occluding the canaliculus as well as a relatively low G' elastic modulus. The combination of relatively low G' elastic modulus and relatively high cohesivity allows less irriation and improved local tolerance as well as longer lasting, typically up to 6 months.

In a particular aspect, the hyaluronic acid (HA) components of a compositon are selected or formulated to provide a compositon that exhibits high cohesivity (e.g. at least 0.3 N according to the compression force method) while also exhibiting a relatively low G' elastic modulus (e.g. G' elastic modulus of less than 400 Pa).

It has been found that such compositions with higher cohesivity and lower G' elastic modulus can provide inter alia favorable application characteristics particularly via injection as well as long-lasting therapeutic benefit for treatment of dry eye syndrome or alleviating symptoms of dry eye syndrome.

In one aspect, a composition for ocular administration is provided comprising a therapeutically effective amount of 1) crosslinked hyaluronic acid and 2) uncrosslinked hyaluronic acid, wherein the composition has i) a cohesivity value of at least 3 mg according to the drop weight method and/or a compression force greater than 0.3 N and ii) a G' elastic modulus of less than 400 Pa, preferably between 200 Pa and 400 Pa.

In one embodiment, the HA component is a crosslinked HA gel having no greater than or up to about 1 weight % to about 5 weight %, 8 weight %, 10 weight %, 15 weight %, 20 weight %, 25 weight %, 30 weight %, 40 weight %, or 50 weight % free (uncrosslinked) HA (weight % based on total HA weight (i.e. both crosslinked and uncrosslinked HA components) in a composition. As referred to herein, free or uncrosslinked HA includes truly free or completely uncrosslinked HA as well as lightly crosslinked (e.g. less than 5, 4, 3, 2 or 1 percent weight percent of the free HA material covalently linked to other HA) HA chains and fragments, suitably in soluble form in water.

In certain aspects, an HA composition comprises up to or greater than about 4 weight %, 5 weight %, 10 weight %, for example, up to or greater than about 15 weight %, for example, up to or greater than about 20 weight % free or uncrosslinked HA.

To achieve an HA composition of relatively high cohesivity as disclosed herein, it may be preferred to limit the amount of free or uncrosslinked HA to about 25 or 20 weight %, or 15, 10 or 8 weight % or less based on total weight of HA in a composition.

In certain preferred aspects, a composition is in the form of a gel, including an aqueous gel, for example, where the hyaluronic acid component(s) are formulated with water or an aqeuous composition/buffer.

Hyaluronic acid (HA, also known as hyaluronan) is a polysaccharide (specifically a glycosaminoglycan) that comprises of repeating D-glucuronic acid and D-N-acetylglucosamine disaccharide units. In certain embodiments, the carboxyl group (-COOH) of D-glucuronic acid may be present in a salt form, e.g. as a sodium salt. These disaccharide units can be viewed in a broader context as "monomers", small molecules that chemically bond to other identical or different monomers to form a macromolecule, or "polymer". The disaccharide monomers that constitute the HA polymer are linked together into a linear chain through beta-1,4 glycosidic bonds. Each disaccharide monomer has a molecular weight of 400 Da. The number of repeating disaccharides, n, in a HA polymer chain can reach 25,000 or more, creating a polymer with a total molecular weight of 10 MDa.

The term "hyaluronic acid" or "HA" as referred to herein designates hyaluronic acid but also physiologically acceptable salts thereof such as sodium, potassium, zinc or silver salts. In certain aspects, a composition may comprise a sodium hyaluronate salt.

In certain aspects, hyaluronic acid used in a present composition suitably has a molecular weight ranging from 1 MDa to 10 MDa, more preferably between 5.10⁴ and 10⁷ Da, still more preferably a molecular weight between 10⁵ and 5.10⁶ Da. After crosslinking and purification, the amount of free HA is suitably between about 5 weight % and 30 weight % based on total weight (both crosslinked and uncrosslinked), more preferably between 10% and 20%, more preferably about 15% . The molecular weight of the free (uncrosslinked) HA is advantageously between about 50,000Da and 1,000,000Da, more preferably between about 100,000Da and 500,000Da.

HA derived from either bacterial or animal sources, generally provided in dry powder form, is a highly water-soluble polymer that will readily dissolve into water to form a viscous clear liquid. The more HA powder added to a given amount of water, the thicker and more viscous the solution will become. Such a solution is known as free HA, uncrosslinked HA, or non-modified HA. If this solution was to be used without crosslinking, the product would be rapidly eliminated from the site of application. In order to overcome the lack of persistence of uncrosslinked HA, crosslinkers are used which bind HA polymer chains to each other, creating a polymer "network" and transforming the viscous liquid into a gel.

According to one embodiment, hyaluronic acid used for treating diseases of the eye, especially dry eye syndrome, comprises 1) crosslinked HA and 2) free or uncrosslinked HA, which has been discovered to improve clinical outcomes when used as a canaliculus occlusive device compared to a device containing no free (uncrosslinked) HA.

Examples of crosslinkers include e.g. 1,4-butanediol diglycidyl ether (BDDE), 1,4-bis(2,3-epoxypropoxy)butane, 1,4-bisglycidyloxybutane, 1,2-bis(2,3-epoxypropoxy) ethylene and 1-(2,3-epoxypropyl)-2,3-epoxycyclohexane, and and di-vinyl sulfone (DVS).

The degree of crosslinking indicates the percentage of HA disaccharide monomer units that are bound to a crosslinker molecule. Every other parameter being equal, the greater the degree of crosslinking, the harder the gel becomes. Also, a higher degree of crosslinking should translate into longer persistence of the HA gel in the body. However, a very high degree of crosslinking might reduce the hydrophilicity (water affinity) of the HA polymer chains and affect the biocompatibility of the product and then induce an immune reaction by the body to the injected HA gel.

Gel hardness, quantified by the variable G' or G' elastic modulus, the elastic or storage modulus, refers to the stiffness of the HA gel, namely its resistance to being deformed. Gel hardness can be illustrated as follows: if a HA gel is placed between two plates and then the upper plate is quickly displaced horizontally a small distance while keeping the lower plate stationary, a force will be required to hold the (shear) deformation in place. G' is defined as the ratio of the shear stress (the force per unit area of plate) to the shear strain imposed (the ratio of the horizontal displacement to the vertical distance between plates). Generally, when the degree of crosslinking is increased, at the same HA concentration, the network formed by the HA polymers becomes more tightly connected, and a greater force is required to displace the gel, and the gel hardness or G' increases.

As shown in the examples, it has been identified that HA gels having a lower G' value are not valuable or less valuable candidates as lacrimal plugs. It has now been found that the most potent HA gels for said use are those having high cohesivity.

As referred to herein, the terms "cohesivity", "cohesion" and "cohesive property" are used interchangeably.

Cohesion is described as the force between particles of the same substance that acts to unite them. In other words, it is easy to separate the particles in a low-cohesive material but more difficult in a material with high cohesion. In IUPACs Golden book, the definition of cohesion is: The work of cohesion per unit area of a single pure liquid or solid phase α,w^{α}_{c} is the work done on the system when a column α of unit area is split, reversibly, normal to the axis of the column to form 2 new surfaces each of unit area in contact with the equilibrium gas phase.

There are several methods for cohesivity measurement, including perceived cohesion, compression force, dispersion in water and drop weight.

According to a preferred embodiment, the cohesivity of the HA gel is measured with the drop weight method. The drop weight method is defined herein by the following protocol: the test is performed as disclosed in the examples below, i.e. in the following conditions: extrusion speed 5 mm/min, with a 30G ½ needle in a 4.77 mm diameter plastic syringe (1ML SPP, CD0424). Several drops (e.g., 25 drops) are collected and the average weight per drop is calculated, advantageously based on 3 distinct experiments.

Alternatively, the gel is filled in a 1 mL BD glass syringe (Becton, Dickinson and Company) and the air was removed by centrifugation. An 18 G cannula with a plane orifice (Intramedic Luer-Stub Adapter 18 G; Becton, Dickinson and Company) is mounted on the syringe and with the use of a Zwick material tester, Zwick BTC-FR 2.5 TH.D09 (Zwick Roell), the gel is extruded at a constant speed of 7.5 mm per minute. When a constant force is achieved, 10 fragments/drops are collected and weighed, and the average weight is calculated.

According to a preferred embodiment, a HA gel as a present composition provides a cohesion value as measured by the drop weight method greater than 3.5 mg, more preferably equal to or greater than 4 mg, 4.5 mg or 5 mg, or even equal to or greater than 5.5 mg. In certain aspects, an HA compositon has a cohesion value of at least 2.0 or 2.5 or 3.0 mg as measured by the drop weight method. In certain aspects the cohesion value is at most 6 mg, or at most 5.5 mg, as measured by the drop weight method.

According to an alternative embodiment, the cohesivity of the HA gel is evaluated by the measurement of the compression force, also referred to as compressive force.

As referred to herein, the compression force (or compressive force) is measured as disclosed in the examples below, i.e. in the following conditions: using a rheometer having a 20 mm plane/plane geometry in an air gap ranging from 2.5 mm to 0.9 mm at a speed of 0.1. mm/sec at room temperature (25° C).

Alternatively, the measurements of the force during compression can be made using an ARES G2 (TA-instruments, Newcastle, DE) equipped with a 25 mm plate-plate measuring geometry at a temperature of 25.0°C. A 1 mL sample is centered on the lower plate. After loading the sample, the upper plate is lowered to a position of 2.5 mm above the lower plate and the sample is allowed to relax for 120 seconds. The force is then measured every second while lowering the upper plate at a rate of 0.0133 mm/s to reach a 0.9 mm gap after 2 minutes.

According to a preferred embodiment, a HA gel to be used as a present composition provides a compression force greater than 0.3 N, more preferably a compression force equal to or greater than 0.4 N, 0.45 N, 0.5N, 0.55N, 0.6 N, 0.65 N, 0.7N or 0.75N, or even equal to or greater than 0.8N or 0.85 N. In certain aspects, an HA composition has a compression force of at least 1.5 or 2.0 or 2.5 N. In certain aspects, an HA composition has a compression force of less than 0.85 N, or less than 0.8 N.

In certain aspects, an HA compositon as disclosed herein with a high cohesivity can be one having:
a value according to the drop weight method greater than 3.5 mg, preferably equal to or greater than 4 mg; or
a value according to the compression force method greater than 0.3 N, preferably equal to or greater than 0.4 N; or
a value according to the drop weight method greater than 3.5 mg, preferably equal to or greater than 4 mg, and a value according to the compression force method greater than 0.3 N, preferably equal to or greater than 0.4 N.

As shown herein, if the hyaluronic acid compositon has such a high cohesivity, it has been found that it is possible to use HA compositions having a lower G' value.

As referred to herein, the G' elastic modulus is measured as disclosed in the examples below, i.e. in the following conditions: with a Kinexus Pro rheometer using a 40 mm plane/plane geometry, with a gap of 250 µm at room temperature (25° C). A 30-minute period is used for relaxation of the sample between loading and measuring. The linear viscoelasticity range is determined by performing a 1Hz fixed frequency amplitude sweep. The modulus G' is determined from the curve obtained in the linear viscoelastic domain where the stress and the displacement are linearly related.

Alternatively, the rheological properties are measured in a frequency sweep from 10 to 0.1 Hz at 0.1% strain (the strain is within the linear viscoelastic region). The measurements are made using an Anton Paar MCR 301 (Anton Paar, Graz, Austria) equipped with a PP-25 measuring system with a gap of 1mm at 25°C. A 30-minute period was used for relaxation of the sample between loading and measuring.

According to a preferred embodiment, an HA composition as disclosed herein has a G' elastic modulus value less than 400 Pa, preferably less than 350 Pa, advantageously substantially equal to 300 Pa. According to another embodiment, an HA composition as disclosed herein has a G' elastic modulus value less than 250 Pa, 200 Pa or 150 Pa, or even less or equal to 100 Pa. In certain aspects, an HA compositon has a G' elastic modulus value of at least 25 Pa, 50 Pa or 75 Pa or 100 Pa or 150 Pa. It is preferred the HA composition has a G' elastic modulus value of at least 200 Pa, and in certain aspects preferably at least 250 Pa. In one embodiment the G' elestic modulus is between 200 Pa and 400 Pa.

According to a specific embodiment, the concentration of hyaluronic acid in the gel is comprised between 1 mg/ml and 100 mg/ml, advantageously between 10 mg/ml and 30 mg/ml, more advantageously between 15 mg/ml and 25 mg/ml, for example 20 mg/ml.

According to another embodiment, the HA gel can contain other active ingredients. Of special interest are anesthetic agents such as lidocaine or a salt thereof, e.g. lidocaine hydrochloride. In relation to lidocaine or lidocaine hydrochloride, a suitable concentration in the HA gel can be comprised between 1 mg/ml and 5 mg/ml, e.g. 3 mg/ml. Alternatively, the HA gel can serve for the delivery of medications, i.e. drugs or combination thereof for treating a predetermined condition (e.g. glaucoma, allergies, infections), impregnated or embedded therein.

As shown in the examples below, a material suitable for use as a present HA composition has a G' of about 300 Pa.

According to a specific embodiment, the concentration of hyaluronic acid in the gel is comprised between 1 mg/ml and 100 mg/ml, advantageously between 10 mg/ml and 30 mg/ml, more advantageously between 15 mg/ml and 25 mg/ml, for example 20 mg/ml.

According to another embodiment, the HA gel can contain other active ingredients. The HA gel can serve for the delivery of medications, i.e. drugs or combination thereof for treating a predetermined condition (e.g. glaucoma, allergies, infections), impregnated or embedded therein.

According to a further aspect, the present invention concerns the use of a crosslinked hyaluronic acid gel containing free HA as a punctal plug, including advantageously a canalicular or intracanalicular plug.

In that context and since the crosslinked hyaluronic acid gel is to be introduced or injected into the body, it can be placed in an injection device, suitably in a syringe, more including in a single-use syringe. In preferred aspects, the HA composition is sterilized before injection, as such or together with the injection device.

As referred to herein, the terms "(intra)canalicular filler", "punctal plug", "punctal occluder", "canalicular occlusive device" are used interchangeably. Such plugs or occluders are intended to slow down the drainage of the tears through the lacrimal ducts by blocking the drain. The lacrimal outflow system is a series of tubes that serve to remove tears from the eye through the nose. Initially, tears drain from the eye through the upper and lower punctal openings, which lead into the canalicular canals, and ultimately to the lacrimal sac. It is in the lacrimal outflow system that drainage of tears from the eye can be adjusted. Also the terms lacrimal filler, gel or occluder may be used interchangeably as the case may be.

As referred to herein, the terms "HA composition" (including a compositon comprising both crosslinked HA and free or uncrosslinked HA components), Lacrimal filler, gel or occuluder are used interchangeably.

According to one embodiment, the hyaluronic acid gel is introduced or placed, advantageously injected, in the canaliculus, advantageously in the lower lid canaliculus as disclosed by Fezza (Clinical Ophthalmology 2018: 12, 2277-2283).

Unexpectedly, it has been discovered that administration of the present HA compoisitons into the lower punctum and canaliculus results in the composition occluding the upper canaliculus.

In particular aspects, the present compositions can be used in the treatment of dry eye syndrome (DES) and/or keratitis sicca (or keratoconjunctivitis sicca) in suitable subjects (e.g. a subject identified in need of such treatment) including mammals and in particular humans. Clinical signs or symptoms of a subject in need of such treatment include red eyes, decreased tear film and corneal epithelial keratopathy on slit lamp exam. The diagnosis and follow-up of dry eyes and keratitis sicca are usually based on the following observations:
- measurement of the tear film break-up (TBUT or TFB, i.e., the time between the blink and the appearance of the first corneal dry spot appearing);
- the staining of the cornea with fluorescein or Rose Bengal and a slit lamp examination (SLE);
- a Schirmer's test wherein a strip of filter paper is placed on the eye hanging over the lower lid to wick the tears.

After introduction of the canalicular occlusive device that comprises an HA composition as disclosed herein such symptoms, especially dry eye can be notably improved (includinge by patient examiantion and/or patient response to questions) for at least 1 month, 3 months, 4 months, 5 months or even 6 months or more after initial treatmentwith an HA compositon and without subsequent treatment of the HA compositon after the intial treatment period. The improvement of the condition can be assessed using at least one of the observations mentioned above.

According to one embodiment, an HA composition as diclosed herein and method for treating dry eyes are provided, comprising the steps of injecting into a canaliculus in a lacrimal outflow system, a HA compositon or gel displaying high cohesivity as disclosed above.

According to another embodiment, the present invention concerns a composition and method for treating dry eyes, comprising the steps of injecting into a canaliculus in a lacrimal outflow system, a crosslinked HA gel containing free HA as disclosed above.

According to a specific embodiment, the method comprises:
- inserting an injector through a punctum and into a canaliculus in a lacrimal outflow system;
- inserting the hyaluronic acid gel occluder in a gel form (i.e., an HA composition in gel form), as disclosed above, from the injector and into the canaliculus, and
- preventing the outflow of liquid through the lacrimal out-flow system with the occluder to retain tears within the eye to maintain eye lubrication and wetness.

The puncta are small orifices on the upper and lower lid margins, measuring 0.5-1.5 mm in diameter. The canaliculi are essentially cylindrical tubes that dilate up to 2 mm in diameter. They are lined by stratified, non-keratinized squamous epithelium, as is the lacrimal sac. The canaliculi are each typically 8-12 mm long. They join to form the single common canaliculus that connects to the lacrimal sac.

According to one embodiment, the volume of the HA gel inserted or injected is comprised between 0.1 mL and 1 mL, advantageously between 0.1 mL and 0.5 mL, e.g. 0.2 mL.

According to another embodiment, the HA gel is inserted once into the lower lid puncta and canaliculus with a syringe and lacrimal irrigator.

The HA gel insertion procedure can contain any one or all the following steps:
- placing one or more drops of an anesthetic agent, e.g., proparacaine, on the surface of the eye;
- stretching the lower lid puncta with a punctal dilator;
- introducing into the lower puncta a lacrimal irrigator with saline, to flush the tear ducts to confirm the patency of the lacrimal outflow system;
- introducing the HA gel into one or both lower lid canaliculus, e.g., using a suitable (e.g., 23-gauge) lacrimal irrigator.

The average time for such a whole procedure is suitably less than 10, 9, 8, 7, 6 or 5 minutes.

Because of the physical properties of the crosslinked HA composition (preferably in gel form), the composition may be easily removed from the canaliculus by simple irrigation with saline solution. If needed, the crosslinked HA composition also can be dissolved or degraded using suitable enzymes, especially hyaluronidases. Similarly to the insertion of the HA composition, the enzyme can be introduced into the canaliculus with a lacrimal catheter and syringe. Thus, the present compositions for treating dry eye can be effectively reversible.

In a further aspect a kit for treating dry eyes is provided comprising: 1) a delivery device such as a syringe; and 2) a crosslinked HA compositon preferably further containing free or uncrosslinked HA as disclosed herein.

In a yet further asepct, a kit for treating dry eyes is provided comprising: 1) delivery device such as a syringe; and 2) a HA gel displaying high cohesivity as disclosed herein.

According to a specific embodiment, the delivery device (e.g. syringe) is adapted to inject a volume of the HA gel comprised between 0.1 mL and 1 mL, advantageously between 0.1 mL and 0.5 mL, e.g. 0.2 mL.

In particular aspects, an injection device (e.g. syringe) have a relatively small size (diameter) can be effectively used, including an injection device (e.g. syringe) having an inner diameter (i.d.) of up to or less than 5, 4.5, 4, 3, 3.5 or 3.2 mm. Use of such a smaller gauge injection device (e.g. syringe) can be a significant benefit to the medical practiitioner administering an HA compositon as disclosed herein to a subject'e eye for treatment of dry eye syndrome or other ocular disease or disorder. The HA composition of the invention allows filling syringes of smaller diameters, which then allow easy extrusion of the product through the needle.

The kit can further contain a lacrimal injector, which is usually similar to conventional lacrimal irrigators. It is advantageously configured to inject said HA gel into a canaliculus in the lacrimal outflow system to absorb liquid within the canaliculus and swell to conform the outer surface of the mass to the inner surface of the canaliculus to form a sealing mechanism for sealing the canaliculus at least partially. Preferably, the sealing mechanism outer surface is sufficiently soft relative to the inner surface of the canaliculus to prevent the sealing mechanism outer surface from eroding the canaliculus lining, and said sealing mechanism forms an occlusion to prevent the outflow of liquid through the lacrimal outflow system to retain tears within the eye to maintain eye lubrication and wetness.

Preferred kits suitably may further include a lacrimal catheter, a punctal dilator and/or enzymes for dissolving the hyaluronic acid mass.

In certain aspects, the present methods of treatment and therapeutic uses may include subjects with one or more of the following identified characteristics (inclusion criteria):
1. A certain specified age such as twenty-two (22) years of age or older;
2. Had a Best-Corrected Distance Visual Acuity (BCDVA) of 20/40 or better in each eye;
3. Had both eyes with a baseline Schirmer's test with anesthetic ≤ 10 mm/5 minutes;
4. Presence of corneal staining in both eyes in any of the 5 areas defined by the NEI/Industry Workshop Scale;
5. Reported a baseline OSDI score of at least 23 with no more than 3 responses of "not applicable";
6. Patent bilateral lacrimal drainage system as demonstrated by punctal irrigation;
7. If female, subject must had been post-menopausal, medically sterile (e.g hysterectomy, bilateral tubal ligation, bilateral oophorectomy) or had negative urine pregnancy test (UPT) on Day 0 of the study and agree to utilize a medically acceptable form of contraception over the course of the study;
8. Have been able and willing to sign informed consent and HIPAA authorization, follow study instructions, and complete all study visits.

In aspects, one or more of any of the above inclusion criteria suitably may be presented on a label, or instructions for use or otherwise associated with a kit or pharmaceutical composition or other delivery package, system or presentation of an HA composition as discussed herein.

In certain aspects, the present methods of treatment and therapeutic uses may exclude subjects with one or more of the following identified characteristics (exclusion criteria):

### Exclusion Criteria:

1. Had use of ophthalmic cyclosporine (Restasis^{®}) within 6 months or lifitegrast (Xiidra^{®}) within 3 months prior to Day 0 in either eye;
2. Had history of surgical punctal occlusion (e.g., cautery), canalicular infection or canalicular surgery in either eye;
3. Had corneal transplant in either eye;
4. Had an ocular surgery (such as cataract surgery or laser in-situ keratomileusis [LASIK]) in either eye within six months of the Baseline Visit;
5. Had a systemic condition or disease not stabilized or judged by the investigator to be incompatible with participation in the study (e.g., current systemic infection, uncontrolled autoimmune disease, uncontrolled immunodeficiency disease);
6. Had history or presence of any ocular disorder or condition in either eye that, in the opinion of the investigator, would interfere with the interpretation of the study results (e.g., significant corneal or conjunctival scarring, pterygium or nodular pinguecula; current ocular infection (except mild blepharitis), conjunctivitis or inflammation not associated with dry eye; anterior (epithelial) basement membrane corneal dystrophy or other clinically significant corneal dystrophy or degeneration; history of ocular herpetic infection; evidence of keratoconus; lid or lacrimal cancer;
7. Had active severe systemic allergy, seasonal allergies, rhinitis or sinusitis requiring treatment (i.e., antihistamines, decongestants, oral or aerosol steroids);
8. Had used of steroids, including administration by systemic or topical ocular routes (dermatologic steroids not applied to the eyelids and inhaled steroids are allowed)
9. Participated in a clinical trial during the past 30 days;
10. Women who were pregnant, planning a pregnancy, or nursing at study entry.

In aspects, one or more of any of the above exclusion criteria suitably may be presented on a label, or instructions for use or otherwise associated with a kit or pharmaceutical composition or other delivery package, system or presentation of an HA composition as disclosed herein.

Other aspects are disclosed infra.

### BRIEF DESCRIPTION OF THE DRAWINGS

The patent or application file contains at least one drawing executed in color. Copies of this patent or patent application publication with color drawings will be provided by the Office upon request and payment of the necessary fee.
FIG. 1 shows schematically one preferred administration protocol of an HA composition (referred to as Lacrifill gel in FIG. 1);
FIG. 2 shows schematically a further preferred administration protocol of an HA composition;

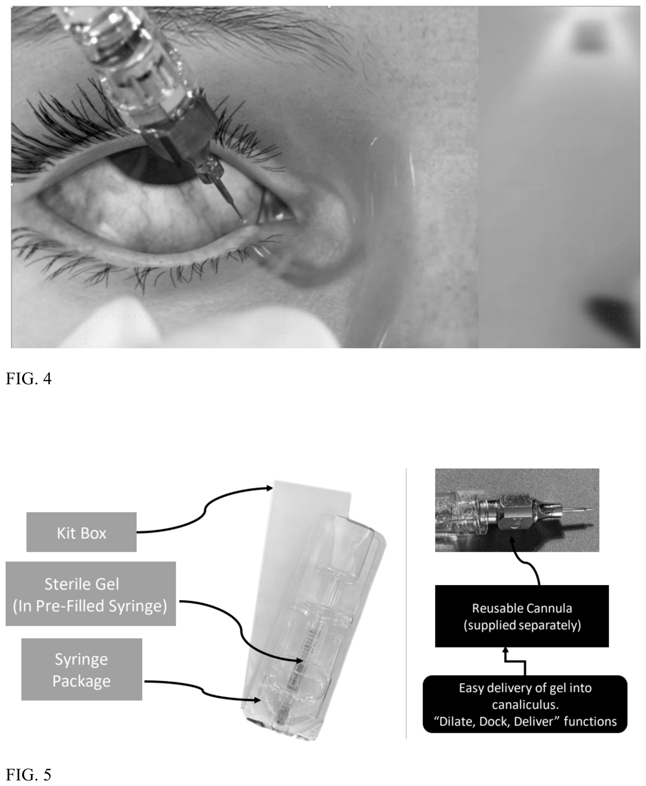

FIGS. 3 and 4 shows an injection to a human subject's eye of an HA composition;
FIG. 5 shows a preferred delivery system and kit for an HA composition (the HA compositopon referred to a s gel in FIG. 5);
FIG. 6 shows a preferred production sequence of an HA composition to a completed kit (Lacrifill Filler Kit);
FIGS. 7, 8 and 9 depict graphically results of Example 2 which follows. In each of FIGS. 7, 8 and 9 , the values higher on the y axis are for an HA plug, and the lower values are Oasis;
FIG. 10 shows means change in Schirmer test score (mm) from baseline over time-Intent-to-treat population. SD=standard deviation. *P<0.01, **P<0.001
FIG. 11 shows graphically mean change in total OSDI score (questions 1-12) from baseline over time-Intent-to-treat population. OSDI=ocular surface disease index, SD=standard deviation. **P<0.001
FIG. 12 shows graphically mean change in total corneal fluorescein staining (NEI scale) from baseline over time-Intent-to-treat population. NEI=National Eye Institute, SD=standard deviation. *P<0.01, **P<0.001

### DETAILED DESCRIPTION

As disucssed above, in one aspect, compositions for ocular administration are provided comprising a therapeutically effective amount of 1) crosslinked hyaluronic acid and 2) uncrosslinked hyaluronic acid.

We have found and demonstate in the examples that follow that the preferred present compositons can provide long-lasting efficacy including for treatment of dry eye syndrome symptoms in human patients, for example for up to or more than 1, 2, 3, 4, 5 or 6 months after a single administration protocol. This represents a significant advance in patient care.

In certain preferred aspects, compositions can be predominately, substantially or completely free of other agents other than components hyaluronic acid-based components particularly any additional therapeutic agents. Thus, in one aspect, compositons are provided that consist essentially of therapeutically effective amounts of 1) crosslinked hyaluronic acid and 2) uncrosslinked hyaluronic acid. In a further aspect, compositons are provided that consist of therapeutically effective amounts of 1) crosslinked hyaluronic acid and 2) uncrosslinked hyaluronic acid. The term "consists" or "consists essentially of" does not preclude the presence of water of injection, physiological salt and buffer salts to obtain physiological pH, since those components are usually necessarily present in HA compositions.

In other aspects, compositions are provided that comprise therapeutically effective amounts of 1) crosslinked hyaluronic acid; and 2) uncrosslinked hyaluronic acid; and 3) one or more therapeutic agents distinct from the 1) and 2) hyaluronic acid.

In further preferred aspects, compositons for ocular administation are provided wherein 1) crosslinked hyaluronic acid and 2) uncrosslinked hyaluronic acid are selected to provide a of at least 0.3 N according to the compression force method.

In further preferred aspects, compositions for ocular administration provided that comprise a therapeutically effective amount of hyaluronic acid, wherein the composition has a cohesivity value of at least 3 mg according to the drop weight method and/or a cohesivity value of at least 0.3 N according to the compression force method. In aspects, such a compositoon suitably has a cohesivity value of at least 3.5 mg according to the drop weight method, or a cohesivity value of at least 4 mg according to the drop weight method, or a cohesivity value of at least 0.4 N mg according to the compression force method. In aspects, suitably such a composition may have a G' elastic modulus between 200 Pa and 400 Pa, and/or a a G' elastic modulus of less than 400 Pa, and/or a G' elastic modulus of less than 350 Pa, or a G' elastic modulus of more than 200 Pa.

In a further preferred aspects, compositions for ocular adminstrations are provided that comprise a therapeutically effective amount of hyaluronic acid, wherein the composition has a has a G' elastic modulus less than 400 Pa. In aspects, such a composition suitably has a G' elastic modulus between 200 Pa and 400 Pa, and/or a G' elastic modulus of less than 350 Pa, and/or a G' elastic modulus of more than 200 Pa. In aspects, such a composition suitably has a cohesivity value of at least 3 mg according to the drop weight method and/or a cohesivity value of at least 0.3 N according to the compression force method. In aspects, such a composition suitably has a cohesivity value of at least 3.5 mg according to the drop weight method, or a cohesivity value of at least 4 mg according to the drop weight method, and/or a cohesivity value of at least 0.4 N according to the compression force method. In aspects, such a composition suitably comprises omprises crosslinked hyaluronic acid.

As discussed, in aspects, preferred compositions exhibit high cohesivity values, for example a cohesivity value of at least 3.5 mg according to the drop weight method (as defined above), or at least 4 mg according to the drop weight method (as defined above). In aspects, a composition as disclosed herein may be formulated where the hyaluronic acid component(s) (e.g. 1) crosslinked hyaluronic acid and 2) uncrosslinked hyaluronic acid) are selected to provide a desired cohesivity value.

In further preferred aspects, compositions for ocular administration are provided comprising a therapeutically effective amount of 1) crosslinked hyaluronic acid and 2) uncrosslinked hyaluronic acid, wherein the composition has i) a cohesivity value of at least 3 mg according to the drop weight method and ii) a G' elastic modulus of less than 400 Pa.

In a further aspects, compositions are provided for ocular administration that comprise a therapeutically effective amount of one or more hyaluronic acid components (e.g. one or more of crosslinked hyaluronic acid and uncrosslinked hyaluronic acid), wherein the composition has a cohesivity value of at least 3.5 mg according to the drop weight method and/or a cohesivity value of at least 0.3 N according to the compression force method. Such compositions also may have a G' value less than 350 Pa.

In further aspects, preferred compositions can exhibit a G' value less than 350 Pa. More particularly, in aspects, compositions are provided for ocular administration that comprise a therapeutically effective amount of one or more hyaluronic acid components (e.g. one or more of crosslinked hyaluronic acid and uncrosslinked hyaluronic acid), wherein the composition has a has a G' value less than 350 Pa.

In further aspects, preferred compositions may have a total concentration of hyaluronic acid components (e.g. both crosslinked and uncrosslinked hyaluronic acid) of between 15 and 25 mg/mL, or 18 and 22 mg/mL. In aspects, a composition as disclosed herein may be formulated where the hyaluronic acid component(s) (e.g. 1) crosslinked hyaluronic acid and 2) uncrosslinked hyaluronic acid) are selected to provide a desired G' value.

In further aspects, preferred compositions may have a total concentration of uncrosslinked hyaluronic acid is present in an amount of between 5 and 30 weight percent based on total weight of hyaluronic acid present in the composition. In certain aspects, preferred compositions may have a total concentration of uncrosslinked hyaluronic acid present in an amount of between 10 and 20 weight percent based on total weight of hyaluronic acid present in the composition.

In aspects, preferred compositions may contain uncrosslinked hyaluronic acid that has a weight average molecular weight of between 50,000Da and 1,000,000Da, or between 100,000Da and 300,000Da.

In aspects, in preferred compositions, crosslinked hyaluronic acid comprises a reacted crosslinker agent, e.g., one or more of 1,4-butanediol diglycidal ether (BDDE) or di-vinyl sulfone (DVS).

In preferred aspects, compositions are in the form of a gel, including an aqueous gel, i.e. comprising water or an aqueous buffer.

In one aspect, the HA composition may be a gel containing crosslinked HA gel and from 1 weight % to 5, 10, 15, 20, 25, 30, 35, 40 or 50 weight percent free or uncrosslinked HA. In certain asepcts, , the HA composition may be a gel containing crosslinked HA gel and from 1 weight % to 5, 10, 15, or 20 weight percent free or uncrosslinked HA.

In another aspect, the crosslinked HA material of an HA composition suitably may be in particle form admixed with free or uncrosslinked HA and suitably in an aqueous gel form.

If present in a particle form, suitably the crosslinked HA particles may have a mean particle size (longest dimension) of e.g. up to or greater than 100 µm, 150 µm, 200 µm, 250 µm, 300 µm or 400 µm. In aspects, the mean particle size (longest dimension) of crosslinked HA particles may be 2500 µm, 2000 µm, 1500 µm, 1200 µm, 1000 µm, 800 µm or 600 µm or less.

In aspects, a present composition can provide a dry eye therapeutic benefit for more than 3 months, or a a dry eye therapeutic benefit for more than 4 or 5 months or a dry eye therapeutic benefit for at least 6 months.

In aspects, a present composition for ocular administration comprises a therapeutically effective amount of 1) crosslinked hyaluronic acid 2) uncrosslinked hyaluronic acid, or consists essentially of therapeutically effective amount of 1) crosslinked hyaluronic acid and 2) uncrosslinked hyaluronic acid, or consists of therapeutically effective amount of 1) crosslinked hyaluronic acid and 2) uncrosslinked hyaluronic acid.

In aspects, a present composition for ocular administration is free of an additional therapeutic agent.

In aspects, a present composition for ocular administration is free of lidocaine.

In aspects, a present composition for ocular administration comprises one or more therapeutic agents in addition to hyaluronic acid.

In aspects, a present composition for ocular administration has a total concentration of hyaluronic acid between 15 and 25 mg/mL, or a total concentration of hyaluronic acid between 18 and 22 mg/mL.

In aspects, a present composition for ocular administration has uncrosslinked hyaluronic acid is present in an amount of between 5 and 30 weight percent based on total weight of hyaluronic acid present in the composition, or uncrosslinked hyaluronic acid is present in an amount of between 10 and 20 weight percent based on total weight of hyaluronic acid present in the composition.

In aspects, in a present composition for ocular administration the weight average molecular weight of the uncrosslinked hyaluronic acid present in the composition is between 50,000Da and 1,000,000Da, or the weight average molecular weight of the uncrosslinked hyaluronic acid present in the composition is between 100,000Da and 300,000Da.

Particularly preferred compositions are injectable, including at room temperature (25°C). A range of canulas suitably may be used for administraiton of the present compositoons including from 25-gauge to 30-gauge, with an inner diameter of from 260 µm to 160 µm.

In further aspects, treatment kits are provided, including kits that comprise 1) a composition as diclosed herein and 2) instructions for use to treat dry eye syndrome. The instructions suitably may be in written form, including as a packaging label. The kit suitably may be in a package form, preferably containing a therapeutically effective amount of a composition as disclosed herein.

In further aspects, treatment methods are provided, including for treating dry eye syndrome in a subject in need thereof comprising administering to the subject a composition as disclosed herein. In certain preferred treatment protocols, the composition is administered in the lower lid canaliculus of the subject's eye.

In preferred systems, administering the composition (including to only the lower lid canaliculus) occludes both the lower and upper canaliculus of the subject's eye.

In preferred protocols, a composition as diclosed herein may be administered into the subject's lower punctum to occlude the lower canaliculus. In preferred systems, the composition is administered into the subject's lower punctum which also occludes the subject's upper canaliculus.

A preferred administration route is by injection.

In certain aspects, a single dose or single administration protocol (e.g. where the protocol is limited to a single day or 1, 2 or 3 days) of compositions as disclosed herein may be utilized. As discussed, preferred compositions can provide long-lasting therapy including relief from dye eye syndrome symptoms following a single dose or single administration protocol (e.g. where the protocol is limited to a single day or 1, 2 or 3 days) such as a therepeutic benefit for up to or more than 3, 4, 5 or 6 months after the single dose or administration protocol.

In further aspects, use of a composition as disclosed herein is provided for treatment of an ocular disease or disorder, including dry eye syndrome.

### Preparation of HA compositions

The present HA compositions can be generally preferred as disclosed in PCT/EP2022/055296 to Symatese and U.S. Patent 9,238,013 with conditions modified or selected to provide the desired cohesivity value and G' elastic modulus. In general, a purified material such as sodium HA in the form of fibers may be provided and admixed in aqeuous soluton with a suitable crosslinking agent such as BDDE. The crosslinking agent may be present e.g. in an amount that is 3, 5, 10, 15 or 20 weight percent or more relative to the weight of the HA material. The aqueous HA material/crosslinking agent admixture may be suitably heated such as to 40-50°C for 0.5, 1, 1.5 or 2 hours or more preferably with agitation. The resulting HA composition may be washed and netrailized. The washed product may be analyzed and tested for cohesivity value and G' elastic modulus and the synthesis protocol modified as needed to provide the desired cohesivity value and G' elastic modulus. For instance, to provide an increased cohesivity value, a greater amount of crosslinker can be utilized and/or the reaction time or temperature can be increased. To provide a reduced G' elastic modulus, a lower amount of crosslinker agent can be utilized, or a less reactive crosslinker can be employed.

### Methods of Treatment

As discussed above, methods and uses are provided for treating a variety of diseases and disorders and particulary an ocular diseases and disorders. In particular, methods and uses are provided for treating and mitigating symptoms of dry eye syndrome or dry eye disease.

In one aspect, these methods and uses may comprise administering to a subject particularly a human patient a HA composition as disclosed herein. The HA composition may be administered in a single dose or over a single treatment protocol e.g. extending over 12, 24, 24, 48 or 72 hours, or other defined period typically less than 7, 6, 5, 4, or 3 days. As demonstrated in the examples which follow, such as a single dose or single administration can provide long-lasting therapeutic benefit including long-lasting alleviation of dry eye syndrome symptoms such as burning in the eye, dry or scratchy feeling in the eye, blurry vision, and/or red eyes.

In certain embodiments, the subject is suffering from dry eye syndrome also known as keratoconjunctivitis sicca (KCS).

Preferred effective doses and amounts of an HA composition are set forth in the examples which follow. For instance, 0.2 mL of a HA compositon as disclosed herein in gel form may be administered via syringe e.g. to a patient's lower punctum. Effective amounts also can be determined empirically for a particular subject, including through in vivo studies.

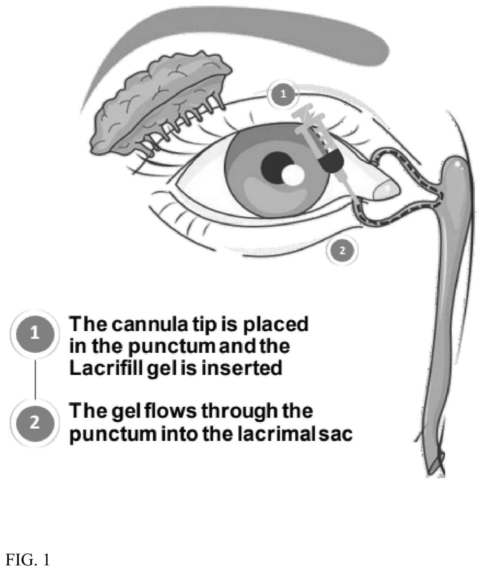

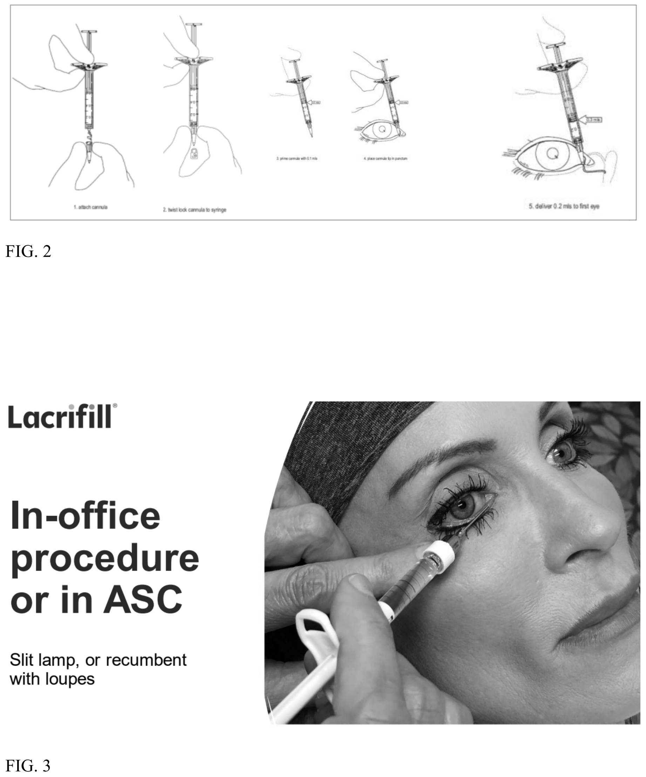

FIGS. 1-3 show preferred administration protocols and application devices.

### Combination Therapy

An HA composition as diclosed herein suitably may be administered to a subject in conjunction or combination with one or more other therapeutic agents, particularly one or more other agents or therapies useful for treatment of dry eye syndrome, or allevation of one or more symptoms of dry eye syndrome.

In one aspect, a subject may receive treatment with a radiopharmaceutical agent as disclosed herein in combination with a regime that can include chemotherapy such as Cyclosporin A (e.g. Restasis^{®}, e.g. loaded onto contact lenses), OCS-02 (Oculis) and/or varenicline (Tyrvaya) (nasal spray) or other agent.

As used herin, the term "in combination" in the context of the administration of a therapy to a subject refers to the use of more than one therapy for therapeutic benefit. The term "in combination" in the context of the administration can also refer to the prophylactic use of a therapy to a subject when used with at least one additional therapy. The use of the term "in combination" does not restrict the order in which the therapies (e.g., a first and second therapy) are administered to a subject. A therapy can be administered prior to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject in need of treatment as disclosed herein. The therapies are administered to a subject in a sequence and within a time interval such that the therapies can act together. In a particular embodiment, the therapies are administered to a subject in a sequence and within a time interval such that they provide an increased benefit than if they were administered otherwise. Any additional therapy can be administered in any order with the other additional therapy.

The Present Application provides the following items :
Item 1. A composition for ocular administration comprising a therapeutically effective amount of 1) crosslinked hyaluronic acid and 2) uncrosslinked hyaluronic acid, wherein the composition has i) a cohesivity value of at least 3 mg according to the drop weight method and ii) a G' elastic modulus of less than 400 Pa.
Item 2. A composition of item 1 wherein the composition can provide a dry eye therapeutic benefit for more than 3 months.
Item 3. A composition of item 1 wherein the composition can provide a dry eye therapeutic benefit for more than 4 or 5 months.
Item 4. A composition of item 1 wherein the composition can provide a dry eye therapeutic benefit for at least 6 months.
Item 5. A composition of any one of items 1 to 4 wherein the composition consists essentially of therapeutically effective amount of 1) crosslinked hyaluronic acid and 2) uncrosslinked hyaluronic acid.
Item 6. A composition of any one of items 1 to 4 wherein the composition consists of therapeutically effective amount of 1) crosslinked hyaluronic acid and 2) uncrosslinked hyaluronic acid.
Item 7. A composition of any one of items 1 to 6 wherein the composition is free of an additional therapeutic agent.
Item 8. A composition of any one of items 1 to 7 wherein the composition is free of lidocaine.
Item 9. A composition of any one of items 1 to 4 wherein the composition comprises one or more therapeutic agents in addition to hyaluronic acid.
Item 10. A composition of any one of items 1 to 9 wherein the composition has a cohesivity value of at least 3.5 mg according to the drop weight method.
Item 11. A composition of any one of items 1 to 9 wherein the composition has a cohesivity value of at least 4 mg according to the drop weight method.
Item 12. A composition of any one of items 1 to 11 wherein the composition has a cohesivity value of at least 0.3 N according to the compression force method.
Item 13. A composition of any one of items 1 to 11 wherein the composition has a cohesivity value of at least 0.4 N according to the compression force method.
Item 14. A composition of any one of items 1 to 13 wherein the composition has a G' elastic modulus of less than 350 Pa.
Item 15. A composition of any one of items 1 to 13 wherein the composition has a G' elastic modulus of up to or more than 200 Pa.
Item 16. A composition of any one of items 1 to 15 wherein the composition has a total concentration of hyaluronic acid between 15 and 25 mg/mL.
Item 17. A composition of any one of items 1 to 15 wherein the composition has a total concentration of hyaluronic acid between 18 and 22 mg/mL.
Item 18. A composition of any one of items 1 to 17 wherein uncrosslinked hyaluronic acid is present in an amount of between 5 and 30 weight percent based on total weight of hyaluronic acid present in the composition.
Item 19. A composition of any one of items 1 to 17 wherein uncrosslinked hyaluronic acid is present in an amount of between 10 and 20 weight percent based on total weight of hyaluronic acid present in the composition.
Item 20. A composition of any one of items 1 to 19 wherein the weight average molecular weight of the uncrosslinked hyaluronic acid is between 50,000D and 1,000,000D.
Item 21. A composition of any one of items 1 to 19 wherein the weight average molecular weight of the uncrosslinked hyaluronic acid is between 100,000Da and 300,000Da.
Item 22. A composition of any one of items 1 to 21 wherein 1) crosslinked hyaluronic acid and 2) uncrosslinked hyaluronic acid are selected to provide a desired cohesivity value.
Item 23. A composition of any one of items 1 to 22 wherein 1) crosslinked hyaluronic acid and 2) uncrosslinked hyaluronic acid are selected to provide a cohesivity value of at least 0.3 N according to the compression force method.
Item 24. A composition of any one of items 1 to 23 wherein 1) crosslinked hyaluronic acid and 2) uncrosslinked hyaluronic acid are selected to provide a desired G' elastic modulus.
Item 25. A composition of any one of items 1 to 24 wherein 1) crosslinked hyaluronic acid and 2) uncrosslinked hyaluronic acid are selected to provide a G' elastic modulus of less than 350 Pa.
Item 26. A composition of any one of items 1 to 25 wherein crosslinked hyaluronic acid comprises a reacted crosslinker agent.
Item 27. A composition of item 26 wherein the crosslinker agent prior to reaction comprises one or more of 1,4-butanediol diglycidal ether (BDDE) or di-vinyl sulfone (DVS).
Item 28. A composition of any one of items 1 to 27 wherein the composition is in the form of a gel.
Item 29. A composition of any one of items 1 to 28 wherein the composition is injectable.
Item 30. An ocular plug comprising a composition of any one of items 1 to 29.
Item 31. The plug of item 30 wherein the plug is a canalicular plug.
Item 32. A kit comprising 1) a composition of any one of items 1 to 29 and 2) instructions for use to treat dry eye syndrome.
Item 33. A kit comprising:
   1) a composition of any one of items 1 to 29;
   2) a syringe.
Item 34. The kit of item 33 further comprising a lacrimal injector.
Item 35. The kit of item 33 or 34 wherein the syringe has inner diameter of less than 5 mm.
Item 36. A method for treating dry eye syndrome in a subject in need thereof comprising:
   administering to the subject a composition of any one of items 1 to 29.
Item 37. A method of item 36 wherein the composition is administered in the lower lid canaliculus of the subject's eye.
Item 38. A method of item 36 or 37 wherein administering the composition occludes both the lower and upper canaliculus of the subject's eye.
Item 39. A method of any one of items 36 to 38 wherein the composition is administered into the subject's lower punctum to occlude the lower canaliculus.
Item 40. A method as in item 39 wherein the composition is administered into the subject's lower punctum which also occludes the subject's upper canaliculus.
Item 41. A method of any one of items 36 to 40 wherein the composition is administered by injection.
Item 42. A method of any one of items 36 to 41 wherein the composition is administered with a syringe having an inner diameter of up to or less than 5, 4.5, 4, 3.8 or 3.5 mm or less.
Item 43. A method of any one of items 36 to 42 wherein the administered composition provides effective dry eye syndrome treatment for more than 3 months following the administration.
Item 44. A method of any one of items 36 to 43 wherein the administered composition provides effective dry eye syndrome treatment for more than 4 months following the administration.
Item 45. A method of any one of items 36 to 44 wherein the administered composition provides effective dry eye syndrome treatment for more than 5 months following the administration.
Item 46. A method of any one of items 36 to 45 wherein the administered composition provides effective dry eye syndrome treatment for 6 or more following the administration.
Item 47. A method of any one of items 36 to 46 wherein a single dose of the composition is administered to the subject.
Item 48. Use of a composition of any one of items 1 to 29 for treatment of dry eye syndrome.
Item 49. Use of item 48 wherein the administered composition provides effective dry eye syndrome treatment for more than 3 months following the administration.

The following non-limiting examples are illustrative.

### Example 1: Preparation of preferred HA composition

The following method of preparation provides a preferred crosslinked HA gel suitable for clinical use:
1. High molecular weight (mean 3 million Daltons) sodium hyaluronate is dispersed in water for injection and NaOH and stirred until a homogeneous solution is produced.
2. The HA solution from above is thoroughly mixed with and aqueous solution of butanediol diglycidyl ether (BDDE) until a homogeneous solution is produced.
3. The solution from 2 is allowed to stand while crosslinking takes place. Alternatively, the solution suitably may be heated to facilitate crosslinking, e.g., from 40-50°C for a time sufficient to provide a desired level of crosslinking.
4. Once the crosslinking reaction is sufficiently complete to provide an admixture containing both crosslinked and uncrosslinked HA, the resulting gel is cut into pieces and hydrated in phosphate buffered saline (PBS) and hydrochloric acid.
5. The gel from 4 is purified by repeated rinsing in PBS.
6. The gel from 5 is degassed and filled into 1ml syringes.
7. The syringes from 6 are sterilized by autoclaving.

Properties of gel produced:

| | |
|---|---|
| Appearance | colorless, transparent gel |
| pH | 7.0 |
| Extrusion force through a 27 gauge, ½ inch needle | 10 Newtons |
| Total HA | 22 mg/ml |
| Residual BDDE | <1.58 ug/g |
| Bacterial endotoxin (LAL test) | <1 EU/g |
| Rheology (G') | 214 Pa |
| Osmolarity | 309 mOsm |
| Uncrosslinked, free HA | 13% of total HA (2.9 mg/ml) |
| MW of free HA | 100-300 kDa |

The HA products tested in this study for cohesivity are shown in Table 1 below:

**Table 1**

| **Product** | **Manufacturer** | **Hyaluronic Acid concentration (mg/mL)** |
|---|---|---|
| Juvederm VOLBELLA | Allergan | 15 |
| Restylane Lidocaine | Q-Med | 20 |
| HA composition as disclosed herein* | | 20 |

| | | |
|---|---|---|
| *This HA composition contained crosslinked HA and uncrosslinked HA, and does not contain Lidocaine. | | |

### G' elastic modulus

The elastic modulus G' was determined with a Kinexus Pro rheometer using a 40 mm plane/plane geometry, with a gap of 250 µm at room temperature (25° C). A 30-minute period was used for relaxation of the sample between loading and measuring. The linear viscoelasticity range is determined by performing a 1Hz fixed frequency amplitude sweep. The modulus G' is determined from the curve obtained in the linear viscoelastic domain where the stress and the displacement are linearly related.

### Compression Force

The linear compressive force (F) was determined using a rheometer having a 20 mm plane/plane geometry in an air gap ranging from 2.5 mm to 0.9 mm at a speed of 0.1. mm/sec at room temperature (25° C).

### Drop weight

This test consists in measuring the weight of a drop of gel extruded out of a defined syringe and canula (the heavier the drop, the more cohesive).

The weight of an average fragment/drop of a HA gel that is pushed through a defined vertical orifice at a constant speed was determined. Briefly, the test conditions were the following: extrusion speed 5 mm/min, with a 30G ½ needle in a 4.77 mm diameter plastic syringe (1ML SPP, CD0424). The weighing of 25 drops was done and the average weight per drop was calculated based on 3 distinct experiments.

### RESULTS OF THE COHESIVITY STUDIES

### Characterization of different hyaluronic acid (HA) gels

The physicochemical properties of the acid hyaluronic gel used by Fezza (Clinical Ophthalmology, 2018: 12, 2277-83), i.e. Restylane-L (where L stands for Lidocaine) (Q-Med) noted x1HA, were compared to other acid hyaluronic based compositions.

Based on the statement that a high degree of reticulation, reflected by a high G' value, could be prejudicial in this context because of possible inflammation, this value was investigated but also the cohesion value, with the assumption that this feature would be the most important.

Therefore, two different tests were performed on said different gels, i.e.
- the measurement of the linear compressive force or compression force (F);
- the measurement of the cohesion by the drop weight method.

The data obtained are shown in Table 2 below:

| **Product** | **G' (Pa)** | **Compression Force (N)** | **Drop weight (mg)** |
|---|---|---|---|
| Juvederm VOLBELLA | 181 | 0.14 | 3.23 |
| Restylane Lidocaine | 524 | 0.25 | 2.71 |
| HA composition as disclosed herein* | 280 | 0.5 | 5.09 |

| | | | |
|---|---|---|---|
| n.d.: not determined. *This HA composition contained crosslinked HA and uncrosslinked HA and does not contain Lidocaine. | | | |

The data shown in Table 2 reveal a good correlation between the value of compression force and the value of drop weight, which constitute valuable methods to evaluate the cohesivity of hyaluronic acid gel fillers.

Moreover, a relationship has been established between cohesion and rheology.

As explained above, in the context of the invention, preferred HA gels are those having high cohesivity, advantageously those having a drop weight value above 4 mg. Interestingly, HA gels selected on that criterion can also display lower G', which is beneficial in terms of putative side effects. The results also indicate that HA gels with low G' are not necessarily compliant with the high cohesivity criterion, see Juvederm VOLBELLA. T he invention provides for HA gels having both low G' and high cohesivity and their use in the treatment of ocular disease including dry eye disease.

### Example 2: Clinical study

A randomized, double-masked, multicenter clinical study approved by the FDA was preformed comparing the gel from above to the OASIS Form fit plug, a polyvinyl pyrrolidone canalicular plug.

Both products significantly improved clinical signs and symptoms of DES as indicated in the tables below:

**Table A. HA plug Improvements in signs and symptoms at 3 and 6 months.**

| | **Baseline** | **3 mos** | **CFB** | **% CHANGE** | **p-value** | **6 mos** | **CFB** | **%CHANGE** | **p-value** |
|---|---|---|---|---|---|---|---|---|---|
| **Mean Schirmer** | 5.4 | 8.7 | 3.4 | 63% | <0.0001 | 8.9 | 3.6 | 67% | <0.0001 |
| **Mean Staining** | 5.9 | 3.9 | -2.1 | -36% | <0.0001 | 3.6 | -2.4 | -41% | <0.0001 |
| **Mean TBUT** | 3.0 | 3.8 | 0.8 | 27% | <0.0001 | 4.0 | 1.0 | 33% | <0.0001 |
| **Mean OSDI** | 48.3 | 21.4 | -27.0 | -56% | <0.0001 | 23.7 | -24.7 | -51% | <0.0001 |

**Table B. Oasis Improvements in signs and symptoms at 3 and 6 months.**

| | **Baseline** | **3 mos** | **CFB** | **% CHANGE** | **p-value** | **6 mos** | **CFB** | **%CHANGE** | **p-value** |
|---|---|---|---|---|---|---|---|---|---|
| **Mean Schirmer** | 5.5 | 7.6 | 2.0 | 36% | 0.0035 | 7.5 | 1.9 | 34% | 0.0007 |
| **Mean Staining** | 5.9 | 3.6 | -2.2 | -37% | <0.0001 | 4.6 | -1.4 | -24% | 0.0045 |
| **Mean TBUT** | 3.1 | 3.6 | 0.5 | 16% | 0.01 | 3.6 | 0.5 | 16% | 0.02 |
| **Mean OSDI** | 45.6 | 19.9 | -26.0 | -57% | <0.0001 | 21.0 | -24.9 | -55% | <0.0001 |

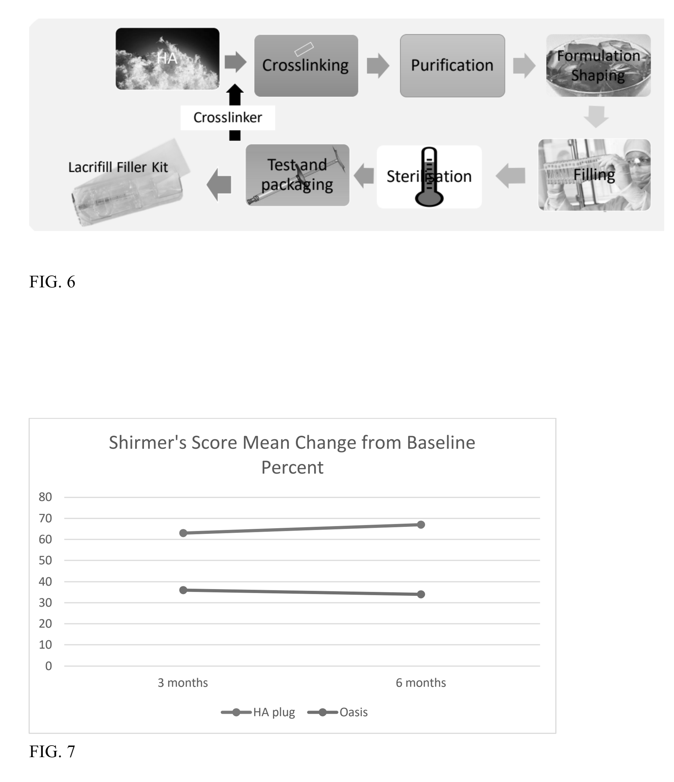

As is seen in the results set forth in FIGS. 7, 8 and 9, the HA product of the present invention performed better than the Oasis plug in those clinical parameters that are indicative of the health of the corneal surface.

In addition, as is seen from Table A, the present HA gel demonstrated effectiveness over a six-month period, with no decrease in effectiveness from month 3 to 6.

In addition, a plug insertion questionnaire was administered upon instillation of the hyaluronic plug into the lower canaliculus. In 33% of cases, gel extrusion from the upper punctum was reported during insertion.

### Example 3

A further description of the clinical study disclosed in Example 2 is provided in this Example 3.

The objective of this study was to evaluate the effectiveness and safety of Lacrifill (an aqeuous gel HA composition as disclosed herein containing both crosslinked and uncrosslinked HA) compared to a commercially available canalicular plug

### Methods

This was a prospective, multicenter, controlled, double-masked, randomized (2:1) study conducted at 5 sites in the United States. The study protocol was reviewed and approved by an Institutional Review Board in accordance with the Declaration of Helsinki and the International Council for Harmonisation Guideline for Good Clinical Practice. All patients provided written informed consent and HIPAA regulations were followed.

Included were adults ≥22 years old with Schirmer test (with anesthesia) ≤10 mm/5 minutes, presence of corneal staining, ocular surface disease index (OSDI) of ≥23 with ≤3 responses of "not applicable," patent lacrimal drainage system, and bilateral best-corrected distance visual acuity (BCDVA) of 20/40 or better. Key exclusion criteria were use of ophthalmic cyclosporine within 6 months or Lifitegrast (Bausch+Lomb) within 3 months prior to Day 0 because of the potential for a confounding effect; history of surgical punctal occlusion, canalicular infection, canalicular surgery, or ocular surgery within 6 months of Day 0; and any ocular or systemic disorder that would interfere with study results.

### Intervention procedure

Lacrifill was instilled bilaterally according to the manufacturer's instructions.¹³ Briefly, following topical anesthesia, the lower puncta was flushed with saline to confirm patency. A cannula attached to a prefilled Lacrifill syringe was inserted into the lower punctum, and 0.2 mL Lacrifill was introduced into each canaliculus. To remove the plug at 6 months, the lower canaliculus was irrigated with sterile saline until it freely flowed through the drainage system and/or the patient reported swallowing or fluid sensation in the nose/throat. Form Fit was placed bilaterally according to the manufacturer's instructions.¹⁴

### Outcome measures and data analysis

Assessments occurred at baseline and Months 3 and 6. The primary effectiveness endpoint was non-inferiority of the mean within subject change from baseline to Month 3 in Schirmer score for patients receiving Lacrifill compared to Form Fit (non-inferiority margin of 2.5 mm). The key secondary effectiveness endpoint was the non-inferiority of the proportion of patients with Lacrifill achieving improvement from baseline to Month 3 in OSDI by a minimal clinically important difference (MCID)¹⁵ of 4.5 points for those with moderate baseline symptoms and 7.3 points for those with severe baseline symptoms compared to patients receiving the Form Fit (noninferiority margin of 20%). Additional secondary endpoints were the mean change from baseline to 3 and 6 months in tear meniscus height (TMH, average of 3 measurements), OSDI (scale 0-100), corneal fluorescein staining (sum of all regions measured via the National Eye Institute scale 0-3), tear break-up time (TBUT, average of 2 measurements), and anesthetized Schirmer score. Safety was assessed by monitoring AEs, BCDVA, biomicroscopy, and IOP. A dye disappearance test evaluated patency on a scale of 0-6, where >3 = occluded and <3 = patent.¹⁶ Investigators rated plug insertion and removal via a questionnaire.

The effectiveness endpoints were tested in a hierarchical fixed sequence of both eyes averaged together to maintain the type I error rate and adjust for multiplicity. Each secondary endpoint was tested at one-sided alpha=0.025. The p-values for secondary endpoints were not interpreted after the first sequential secondary endpoint was found not significant. Any remaining untested secondary endpoints were then considered exploratory. Continuous and categorical variables were summarized with descriptive statistics, frequencies, and percentages. Data were analyzed using SAS (v9.4, SAS Institute) and included linear mixed models with repeated measures, logistic regression models, Fisher's exact test, and two-sample t-tests; significance was P<0.05. To allow for 80% power to reject the primary null hypothesis and a 10% dropout rate, a sample size of 162 patients (108 with Lacrifill and 54 with Form Fit) was targeted for enrollment.

### Results

A total of 157 patients were randomized; 99 of 103 (96.1%) patients with Lacrifill and 52 of 54 (96.3%) patients with Form Fit completed the study. Four patients in the Lacrifill group discontinued (1 lost to follow-up, 1 unable to instill, and 2 noncompliance), and 2 patients in the Form Fit group discontinued (voluntary withdrawal). Both groups had similar demographics and baseline characteristics (Table 1). The majority of women were postmenopausal (64.7%, 101/156). Ocular medical history included meibomian gland dysfunction (19.7%, 31/157), blepharitis (8.3%, 13/157), LASIK (16.6%, 26/157), and cataract surgery (15.9%, 25/157).

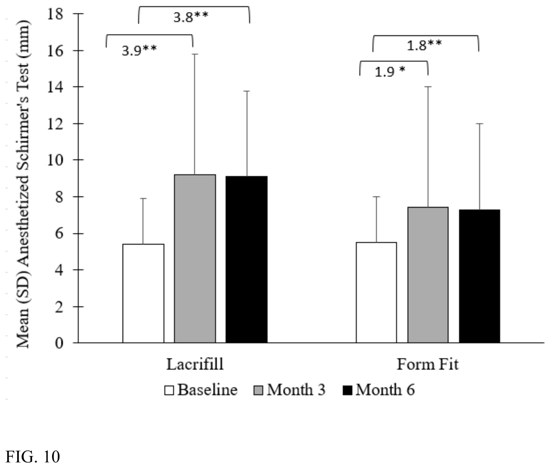

At Month 3, Lacrifill was non-inferior to Form Fit in the Schirmer score change from baseline with a margin of <2.5 mm; meeting the primary endpoint (LSMean difference [two-sided 95%CI]=1.77 [-0.63, 4.18], P=0.0003). Schirmer score significantly improved from baseline to Month 3 and Month 6 in both groups (FIG. 10). The change from baseline was 3.9±7.6 mm (P<0.0001) and 1.9±5.0 mm (P=0.0047) at Month 3, and 3.8±6.7 mm (P<0.0001) and 1.8±4.0 mm (P=0.0009) at Month 6 in the Lacrifill and Form Fit groups, respectively.

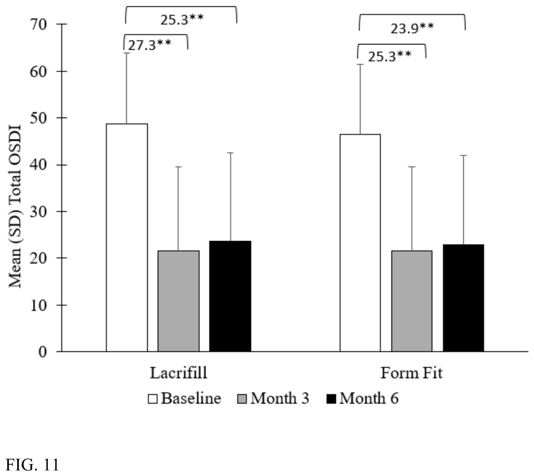

The key secondary endpoint was met, Lacrifill was non-inferior to Form Fit in the proportion of patients achieving improvement from baseline to Month 3 in OSDI score by an MCID (84.3% [86/102] and 83.3% [45/54], respectively, mean difference [two-sided 95%CI]=-0.009 [-0.121, 0.103], P=0.0004). OSDI significantly improved from baseline to Month 3 and Month 6 in both groups (FIG. 11). The change from baseline was 27.3±20.30 (P<0.0001) and 25.3±16.24 (P<0.0001) at Month 3, and 25.3±20.11 (P<0.0001) and 23.9+18.98 (P<0.0001) at Month 6 in the Lacrifill and Form Fit groups, respectively.

### Secondary Endpoints

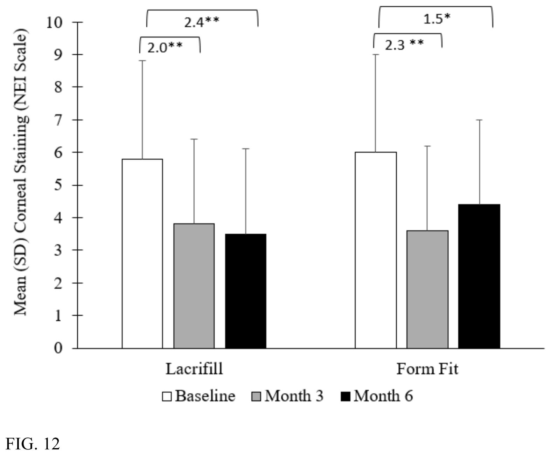

Neither group had a significant change from baseline in TMH; the change from baseline was 0.033+0.6931 (P=0.6837) and 0.002+0.7681 (P=0.5084) at Month 3, and 0.084+0.7951 (P<0.8530) and 0.070+0.8003 (P<0.7354) at Month 6 in the Lacrifill and Form Fit groups, respectively. Corneal staining significantly improved from baseline to Month 3 and Month 6 in both groups (FIG. 12). The change from baseline was 2.0±3.18 (P<0.0001) and 2.3±3.72 (P<0.0001) at Month 3, and 2.4±3.51 (P<0.0001) and 1.5±3.70 (P=0.0024) at Month 6 in the Lacrifill and Form Fit groups, respectively. TBUT significantly improved from baseline to Month 3 and Month 6 in both groups. The change from baseline was 0.787±1.5032 sec (P<0.0001) and 0.787±1.5032 sec (P=0.0063) at Month 3, and 1.054±1.5996 sec (P<0.0001) and 0.531±1.7181 sec (P=0.0151) at Month 6 in the Lacrifill and Form Fit groups, respectively.

### Safety

Both groups had a similar rate of patients with ≥1 ocular device-related AEs (Table 2). No patient discontinued the study due to an AE. There were no ocular serious AEs. Increased corneal staining (reported as an AE) occurred in 7.8% (8/103) of the Lacrifill group and 16.7% (9/54) of the Form Fit group. Neither group had a significant loss of BCDVA, and there was no notable change in IOP. Dye disappearance mean scores increased from baseline and were maintained over the course of the study for both groups (Lacrifill: 2.94 OD and 2.91 OS at baseline, 4.47 OD and 4.54 OS at Month 3, and 4.42 OD and 4.49 OS at Month 6; Form Fit: 2.81 OD and 2.86 OS at baseline, 4.28 OD and 4.28 OS at Month 3, 4.48 OD and 4.46 OS at Month 6).

Both plugs were easy to insert, with no to minimal pain during the procedure (Table 3). In 33.3% of cases, gel extruded from the upper punctum during insertion of Lacrifill. Both Lacrifill and Form Fit plugs were easy to irrigate from the canaliculus, with minimal difficulties, and the patency of the drainage system was restored.

### Discussion

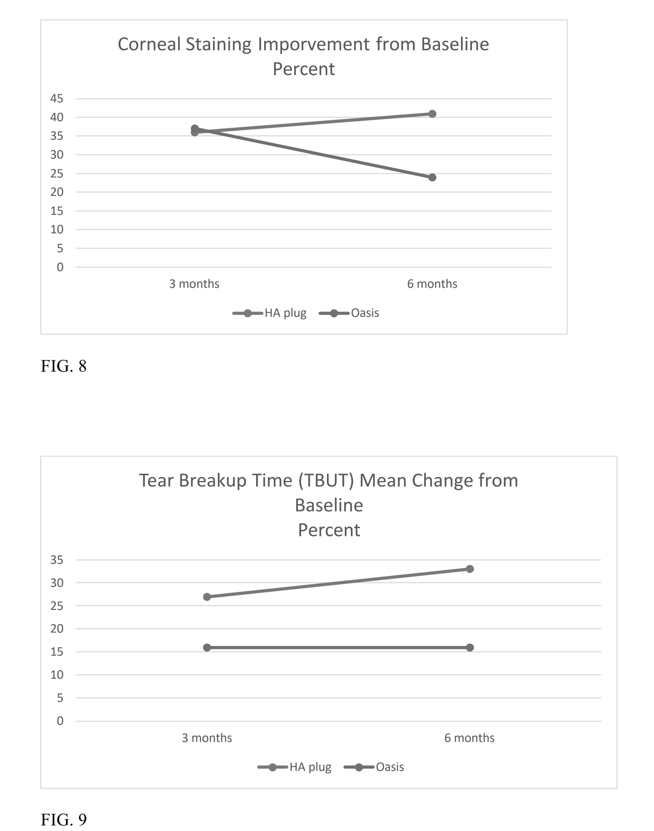

Lacrifill was noninferior to Form Fit in Schirmer score improvement from baseline to 3 months and the proportion of OSDI responders. Compared with the Form Fit group, the Lacrifill group had a trend towards superior increase of Schirmer score at 3 and 6 months (FIG. 7) and had a larger improvement in corneal staining at 6 months (FIG. 9). The improvements in Schirmer score, OSDI, corneal staining, and TBUT were sustained over 6 months. This, along with the dye disappearance, test confirms that Lacrifill was in place for 6 months. Lacrifill was safe, well tolerated, and easy to insert and easy to remove at 6 months. The effectiveness, safety and superiority of Lacrifill are evidenced when considering a proof-of-concept study in 63 patients who received a different crosslinked HA intracanalicular gel (Restylane Lidocaine, Q Med).¹⁷

TFOS DEWS II concluded that creating an adequate environment for ocular surface health can be accomplished by retention of tears via lacrimal occlusive devices.⁷ Studies show that lacrimal occlusive devices can help improve dry eye associated with refractive surgery, contact lens wear, glaucoma, and menopause.¹⁰ Patients with dry eye that has not been satisfactorily treated with lubricating eye drops may benefit from lacrimal drainage occlusion, particularly patients with moderate dry eye.¹⁸ Considering that approximately one-third of patients without pre-existing DED develop dry eye after cataract surgery and contact lens discomfort attributed to dry eye is commonplace, it is anticipated that Lacrifill will be a useful tool for treating these patients.^{2, 19} In addition, Lacrifill did not cause alteration of IOP, which indicates that it may be useful for patients with dry eye and glaucoma.

Although the study concluded noninferiority, Lacrifill has advantages over Form Fit. Lacrifill completely fills the canaliculus up to the punctum, and in 33.3% of cases gel extruded from the upper punctum during insertion. The benefit is that physicians have visual evidence that the punctum has been blocked, and complete blockage prevents tears from becoming stagnant. Permanent intracanalicular plugs (such as the SmartPlug, Form Fit, and Herrick plug) have been associated with a higher risk of pyogenic granuloma and canaliculitis, which in some cases have necessitated canaliculotomy and dacryocystorhinostomy.^{18, 20, 21} The infection has been attributed to stagnant tears, and accumulation of biomaterial.^{22, 23} SmartPlug may not completely fill the space, which can lead to stagnant tears.^{10, 24} Herrick plug can have a stagnant column of tears that fill the column between the plug and punctal opening.¹⁰ In contrast to these intracanalicular plugs, Lacrifill completely fills the canaliculus, and thus may have a better safety profile by design. Also, it is noteworthy that reports of pyogenic granuloma and canaliculitis leading to surgical removal typically occurred several years post-occlusion;^{18, 20, 21} whereas, Lacrifill is indicated for 6 months of use, then removal and replacement for increased safety.

Lacrifill also has advantages over punctal plugs. Punctal plugs extrude from the puncta, which can cause localized discomfort due to abrasion of the corneal and conjunctival surfaces. Lacrifill eliminates the potential for abrasion because there is no plug exposure. Punctal plugs can fall out prematurely. Spontaneous loss of punctal plugs has occurred in up to 60% of cases,⁷ with loss reported in 16-48% of cases 1 to 3 months post-insertion, depending on the punctal plug design.²⁵⁻²⁷ In contrast, Lacrifill completely occludes the canaliculus so it won't spontaneously dislodge. Another advantage is that Lacrifill does not require individualized sizing as needed by punctal plugs.

Neither treatment group had an improvement from baseline in TMH, which may be due to insufficient control of variables that affect tear film characteristics and affect TMH; for example, location of the measurement along the eyelid, fluorescein instillation technique, timing of measurement after instillation of fluorescein, variations in time from blink, room light, temperature, and humidity.^{15, 28} In addition, we used 40x magnification to evaluate TMH, which may have been the limiting factor; at magnification >8x, the patient's movements have a larger influence on the measurements.²⁹ Although TMH is commonly used in clinical practice, these limitations may interfere with assessment outcome and may explain why TMH is not a standard endpoint in pivotal studies of dry eye therapies.³⁰

The study was not powered for superiority. However, the change from baseline in Schirmer test score at 3 and 6 months and corneal staining at 6 months was greater with Lacrifill compared with Form Fit. Despite this study limitation, the study design was robust. It was a prospective multicenter, randomized, controlled, double-masked study that included a large population. The population was 65% postmenopausal, 83% had severe dry eye, and 37% had a history of LASIK and/or cataract operation indicating that Lacrifill may be useful in these populations.

This study demonstrates a novel, safe, well-tolerated, and effective method to treat dry eye. Lacrifill effectively occludes the canaliculi, maintaining lubricating tears on the surface of the eye. There were clinically and statistically significant improvements in both signs and symptoms of dry eye, which were sustained through 6 months, when Lacrifill was irrigated from the lacrimal system and could be replaced. Lacrifill is useful in a wide population of patients with dry eye, including those undergoing cataract and refractive surgery.

### References for Example 3

1 Akpek EK, Amescua G, Farid M, et al. Dry Eye Syndrome Preferred Practice Pattern(R). Ophthalmology 2019; 126: P286-P334.
2 Miura M, Inomata T, Nakamura M, et al. Prevalence and Characteristics of Dry Eye Disease After Cataract Surgery: A Systematic Review and Meta-Analysis. Ophthalmol Ther 2022; 11: 1309-1332.
3 Starr CE, Gupta PK, Farid M, et al. An algorithm for the preoperative diagnosis and treatment of ocular surface disorders. J Cataract Refract Surg 2019; 45: 669-684.
4 Schiffman RM, Walt JG, Jacobsen G, et al. Utility assessment among patients with dry eye disease. Ophthalmology 2003; 110: 1412-1419.
5 Mertzanis P, Abetz L, Rajagopalan K, et al. The relative burden of dry eye in patients' lives: comparisons to a U.S. normative sample. Invest Ophthalmol Vis Sci 2005; 46: 46-50.
6 Miljanovic B, Dana R, Sullivan DA, et al. Impact of dry eye syndrome on vision-related quality of life. American journal of ophthalmology 2007; 143: 409-415.
7 Jones L, Downie LE, Korb D, et al. TFOS DEWS II Management and Therapy Report. The ocular surface 2017; 15: 575-628.
8 Semp DA, Beeson D, Sheppard AL, et al. Artificial Tears: A Systematic Review. Clin Optom (Auckl) 2023; 15: 9-27.
9 Kawahara A. Treatment of Dry Eye Disease (DED) in Asia: Strategies for Short Tear Film Breakup Time-Type DED. Pharmaceutics 2023; 15
10 Jehangir N, Bever G, Mahmood SM, et al. Comprehensive Review of the Literature on Existing Punctal Plugs for the Management of Dry Eye Disease. J Ophthalmol 2016; 2016: 9312340.
11 Hynnekleiv L, Magno M, Vernhardsdottir RR, et al. Hyaluronic acid in the treatment of dry eye disease. Acta Ophthalmol 2022; 100: 844-860.
12 Posarelli C, Passani A, Del Re M, et al. Cross-Linked Hyaluronic Acid as Tear Film Substitute. J Ocul Pharmacol Ther 2019; 35: 381-387.
13 Lacrifill Canalicular Plug Instructions for Use. In: Visant Medical, 2022
14 Form Fit Hydrogel Canalicular Plug Instructions for Use. In: Oasis Medical, 2022
15 Wolffsohn JS, Arita R, Chalmers R, et al. TFOS DEWS II Diagnostic Methodology report. The ocular surface 2017; 15: 539-574.
16 Macri A, Rolando M, Pflugfelder S. A standardized visual scale for evaluation of tear fluorescein clearance. Ophthalmology 2000; 107: 1338-1343.
17 Fezza JP. Cross-linked hyaluronic acid gel occlusive device for the treatment of dry eye syndrome. Clinical ophthalmology 2018; 12: 2277-2283.
18 Marcet MM, Shtein RM, Bradley EA, et al. Safety and Efficacy of Lacrimal Drainage System Plugs for Dry Eye Syndrome: A Report by the American Academy of Ophthalmology. Ophthalmology 2015; 122: 1681-1687.
19 Papas EB, Ciolino JB, Jacobs D, et al. The TFOS International Workshop on Contact Lens Discomfort: report of the management and therapy subcommittee. Invest Ophthalmol Vis Sci 2013; 54: TFOS183-203.
20 Joganathan V, Mehta P, Murray A, et al. Complications of intracanalicular plugs: a case series. Orbit 2010; 29: 271-273.
21 Hill RH, 3rd, Norton SW, Bersani TA. Prevalence of canaliculitis requiring removal of Smart Plugs. Ophthalmic Plast Reconstr Surg 2009; 25: 437-439.
22 Sugita J, Yokoi N, Fullwood NJ, et al. The detection of bacteria and bacterial biofilms in punctal plug holes. Cornea 2001; 20: 362-365.
23 Yokoi N, Okada K, Sugita J, et al. Acute conjunctivitis associated with biofilm formation on a punctal plug. Jpn J Ophthalmol 2000; 44: 559-560.
24 Kojima T, Dogru M, Ishida R, et al. Clinical evaluation of the Smart Plug in the treatment of dry eyes. American journal of ophthalmology 2006; 141: 386-388.
25 Horwath-Winter J, Thaci A, Gruber A, et al. Long-term retention rates and complications of silicone punctal plugs in dry eye. American journal of ophthalmology 2007; 144: 441-444.
26 Sakamoto A, Kitagawa K, Tatami A. Efficacy and retention rate of two types of silicone punctal plugs in patients with and without Sjogren syndrome. Cornea 2004; 23: 249-254.
27 Brissette AR, Mednick ZD, Schweitzer KD, et al. Punctal Plug Retention Rates for the Treatment of Moderate to Severe Dry Eye: A Randomized, Double-Masked, Controlled Clinical Trial. American journal of ophthalmology 2015; 160: 238-242 e231.
28 Garcia-Resua C, Santodomingo-Rubido J, Lira M, et al. Clinical assessment of the lower tear meniscus height. Ophthalmic Physiol Opt 2009; 29: 487-496.
29 Imamura H, Tabuchi H, Nakakura S, et al. Usability and reproducibility of tear meniscus values generated via swept-source optical coherence tomography and the slit lamp with a graticule method. Int Ophthalmol 2018; 38: 679-686.
30 Dry Eye: Developing Drugs for Treatment Guidance for Industry. In: Research USDoHaHSFaDACfDEa, ed, 2020

## Claims

1. A composition for ocular administration comprising a therapeutically effective amount of 1) crosslinked hyaluronic acid and 2) uncrosslinked hyaluronic acid,
wherein the composition has i) a cohesivity value of at least 3 mg according to the drop weight method and ii) a G' elastic modulus of less than 400 Pa.

2. A composition of claim 1 wherein the composition consists of therapeutically effective amount of 1) crosslinked hyaluronic acid and 2) uncrosslinked hyaluronic acid.

3. A composition of any one of claims 1 to 2 wherein the composition is free of an additional therapeutic agent.

4. A composition of any one of claims 1 to 3 wherein the composition comprises one or more therapeutic agents in addition to hyaluronic acid.

5. A composition of any one of claims 1 to 4 wherein the composition has a cohesivity value of at least 3.5 mg according to the drop weight method, or of at least 4 mg according to the drop weight method.

6. A composition of any one of claims 1 to 5 wherein the composition has a cohesivity value of at least 0.3 N according to the compression force method, or of at least 0.4 N according to the compression force method.

7. A composition of any one of claims 1 to 6 wherein the composition has a G' elastic modulus of less than 350 Pa or of up to or more than 200 Pa.

8. A composition of any one of claims 1 to 7 wherein the composition has a total concentration of hyaluronic acid between 15 and 25 mg/mL or between 18 and 22 mg/mL.

9. A composition of any one of claims 1 to 8 wherein uncrosslinked hyaluronic acid is present in an amount of between 5 and 30 weight percent based on total weight of hyaluronic acid present in the composition or between 10 and 20 weight percent based on total weight of hyaluronic acid present in the composition.

10. A composition of any one of claims 1 to 9 wherein the weight average molecular weight of the uncrosslinked hyaluronic acid is between 50,000D and 1,000,000D or between 100,000Da and 300,000Da.

11. A composition of any one of claims 1 to 10 wherein crosslinked hyaluronic acid comprises a reacted crosslinker agent, preferably the crosslinker agent prior to reaction comprises one or more of 1,4-butanediol diglycidal ether (BDDE) or di-vinyl sulfone (DVS).

12. A composition of any one of claims 1 to 11 wherein the composition is in the form of a gel.

13. A composition of any one of claims 1 to 12 wherein the composition is injectable.

14. An ocular plug comprising a composition of any one of claims 1 to 13.

15. A kit comprising:
1) a composition of any one of claims 1 to 14;
2) a syringe.
